# EUROPEAN PATENT SPECIFICATION

(11) **EP 1 624 899 B1**
(45) Date of publication and mention of the grant of the patent: **17.11.2010**
(21) Application number: 04760720.5
(22) Date of filing: 04.05.2004
(51) Int. Cl.: A61K 48/00, C12N 5/10, C12N 15/63, C12N 15/867, C12N 15/87, C12Q 1/68, C12Q 1/70

(54) **INCREASED TRANSDUCTION USING ABC TRANSPORTER SUBSTRATES that are INHIBITORS**
ERHÖHTE TRANSDUKTION MIT ABC-TRANSPORTERSUBSTRATENHEMMERN
AMELIORATION DE LA TRANSDUCTION AU MOYEN DE SUBSTRATS qui sont INHIBITEURS DU TRANSPORTEUR ABC

(30) Priority: 05.05.2003 US 468207 P
(43) Date of publication of application: 15.02.2006
(73) Proprietor: VIRxSYS Corporation, Gaithersburg, MD 20877 (US)
(72) Inventor: DAVIS, Brian, Albany, NY 12208 (US); HUMEAU, Laurent, Germantown, MD 20876 (US); DROPULIC, Boro, Ellicott City, MD 21042 (US)
(74) Representative: Roques, Sarah Elizabeth
(86) International application number: PCT/US2004/013968
(87) International publication number: WO 2004/098531

(56) References cited:
- WO-A-96/09400
- US-A- 5 977 067
- US-B1- 6 251 629
- HARBISON M A ET AL: "EFFECT OF THE CALCIUM CHANNEL BLOCKER VERAPAMIL ON HUMAN IMMUNODEFICIENCY VIRUS TYPE 1 REPLICATION IN LYMPHOID CELLS" JOURNAL OF INFECTIOUS DISEASES, CHICAGO, IL, US, vol. 164, no. 1, 1 July 1991 (1991-07-01), pages 53-60, XP000568459 ISSN: 0022-1899
- DAVIS BRIAN M ET AL: "ABC transporter inhibitors that are substrates enhance lentiviral vector transduction into primitive hematopoietic progenitor cells." BLOOD. 15 JUL 2004, vol. 104, no. 2, 15 July 2004 (2004-07-15), pages 364-373, XP007900181 ISSN: 0006-4971 & DAVIS BRIAN M ET AL: "ABC transporter substrates improve lentiviral vector transduction into hematopoietic stem cells." BLOOD, vol. 102, no. 11, 16 November 2003 (2003-11-16), page 249a, XP001237234 & 45TH ANNUAL MEETING OF THE AMERICAN SOCIETY OF HEMATOLOGY; SAN DIEGO, CA, USA; DECEMBER 06-09, 2003 ISSN: 0006-4971
- COSTELLO E ET AL: "Gene transfer into stimulated and unstimulated T lymphocytes by HIV-1-derived lentiviral vectors." GENE THERAPY. APR 2000, vol. 7, no. 7, April 2000 (2000-04), pages 596-604, XP001057677 ISSN: 0969-7128
- GALIPEAU J. ET AL.: 'A bicistronic retroviral vector for protecting hematopoietic cells against antifolates and P-glycoprotein effluxed drugs' HUMAN GENE THERAPY vol. 8, 10 October 1997, pages 1773 - 1783, XP008044290
- DATABASE MEDLINE [Online] US NATIONAL LIBRARY OF MEDICINE (NLM), BETHESDA, MD, US; December 1999 (1999-12), VARTHAKAVI V ET AL: "Human immunodeficiency virus replication in a primary effusion lymphoma cell line stimulates lytic-phase replication of Kaposi's sarcoma-associated herpesvirus." Database accession no. NLM10559351 & JOURNAL OF VIROLOGY DEC 1999, vol. 73, no. 12, December 1999 (1999-12), pages 10329-10338, ISSN: 0022-538X

## Description

### Technical Field

The present invention relates to the introduction of viral vector borne nucleic acids into cells expressing ABC transporters. The invention provides methods of increasing transduction efficiency of such nucleic acids by contacting the cells with substrates and/or inhibitors of ABC transporters. The invention also provides compositions and kits relating to the practice of the methods as well as methods of identifying ABC transport substrate and inhibitor compounds as increasing transduction efficiency.

### Background Art

Stem cells possess tremendous potential for treatment of many human diseases. Efficient gene transfer into hematopoietic stem cells, derived from the bone marrow (BM), mobilized peripheral blood (mPB) or cord blood (CB), could provide valuable therapies for genetic deficiencies, as well as for cancer and infectious diseases such as HIV infection. Historically, efficient introduction of therapeutic genes into hematopoietic stem cells has been problematic using retroviral vectors because of inefficient stable integration into non-dividing cells.

In comparison, lentiviral based vectors are powerful tools for gene delivery into a broad range of dividing and non-dividing cells. Lentiviral vectors that contain the central polypurine tract (cppt) and central termination sequence (cts) transduce non-dividing cells more efficiently and are excellent candidate vectors for hematopoietic gene therapy. Thus hematopoietic stem cells are prime targets for lentiviral vectors. Previous reports of lentiviral gene transfer into human CD34+ cells describe gene transfer efficiencies between 10% and 60%. SCID repopulating cells (SRC) are exceptional targets for lentivectors, with gene transfer rates frequently greater than 75%. In general, lentivectors transduce CB derived CD34+ cells more efficiently than CD34+ cells from mobilized peripheral blood (mPB) or bone marrow (BM). mPB and BM, perhaps because of their relatively decreased proliferation potential, are more refractory to transduction, and reported transduction efficiencies range from 10% to 55%.

The experimental analyses of lentiviral vector transduction efficiencies, however, were studied using research-level vector production protocols, hereafter referred to as "research grade", in which cells are transduced with whole supernatant with or without vector concentration by centrifugation. The use of vectors produced at a clinical scale is more challenging for a number of reasons. First, production of vector for clinical gene therapy protocols requires stringent manipulation to ensure safety. Second, production of the large vector amounts necessary for clinical level transduction result in reduced titers and gene transfer efficiencies compared to small-scale research grade vector production. It.has been unclear as to how the decreased transduction efficiencies of "clinical grade" vectors can be improved.

The ATP-binding cassette (ABC) transporter family of proteins is well characterized for its ability to efflux a wide range of lipophilic, structurally small molecules and unrelated drugs, particularly chemotherapeutic agents and HIV protease inhibitors. A number of ABC transporters, in particular P-glycoprotein (P-gp), MRP1 and ABCG2 (also known as Brcpl) are expressed strongly on CD34+ cells. See for example Chaudhary et al. (Cell 1991 66(1):85-94). It has been speculated that P-glycoprotein acts as a regulatory protein in primitive hematopoietic cells, as MDR1 transduced primitive cells expanded *ex vivo* acquired a myeloproliferative defect after transplantation into mice. Furthermore, ABCG2 is a molecular determinant of the "side population" (SP) phenotype of hematopoietic cells and may play a role in maintenance of the most primitive and quiescent hematopoietic stem cells. Therefore, ABC transporters appear to have functional importance in the biology of hematopoietic stem cells.

Much work on ABC transporters has involved P-gp, including studies of various compounds based on their effects on P-gp. These compounds have included anticancer drugs and have been classified in part via their effect on the ATPase activity of human P-gp.

The invention described herein is based upon the surprising discovery that substrates and/or inhibitors of ABC transporters can increase the transduction efficiency of retroviral based vectors.
Harbison et al., J. Infect. Disease, 1991, 164: 53-60 describe the effect of the calcium channel blocker verapamil on HIV-1 replication in lymphoid cells.

Citation of the above documents is not intended.as an admission that any of the foregoing is pertinent prior art. All statements as to the date or representation as to the contents of these documents is based on the information available to the applicant and does not constitute any admission as to the correctness of the dates or contents of these documents.

### Disclosure of the Invention

The present invention provides methods, and compositions related thereto, for increasing the transduction efficiency of viral vectors into cells expressing one or more ABC transporters. The increase is mediated by contacting the cells with a substrate and inhibitor of one or more ABC transporters. By "increasing transduction efficiency," it is meant that transduction is increased in the presence of a substrate and inhibitor in comparison to the absence thereof. Thus, in accordance with the present invention, there is provided an *in vitro* or *ex vivo* method for increasing the transduction efficiency of an ATP-binding cassette (ABC) transporter expressing hematopoietic stem cell (HSC) with a vesicular stomatitis virus G (VSV-G) pseudotyped lentiviral vector, said method comprising:
(a) contacting said HSC with at least one compound comprising a substrate and an inhibitor of said ATP-binding cassette (ABC) transporter wherein said compound is not probenecid; and
(b) contacting said cell with said lentiviral vector.

The contacting of cells with a substrate and inhibitor may occur prior to, or simultaneous with, exposure of the cells to a viral vector particle. The viral vector is a VSV-G pseudotyped lentiviral vector. As used herein, the term "viral vector" denotes encapsidated forms of a nucleic acid molecule derived from a virus and used to transfer genetic material into a cell via transduction. The term encompasses viral particles in which the viral vector nucleic acid have been packaged.

We also describe the use of the transduced cells in other applications, including production of useful gene products and proteins by expression of a nucleic acid sequence present in the viral vector nucleic acid. Another application is the introduction of transduced cells into living subjects afflicted, or at risk of being afflicted with a disease. Preferably, this latter application is in the form of *ex vivo* therapy wherein cells are obtained from a subject, transduced according to the invention, and returned to the subject. More preferably, the subject is human. We also describe that the system may be applied *in vivo* whereby an ABC transporter substrate and inhibitor and a viral vector are administered to a subject in accordance with the invention. In this aspect, the viral vector, ABC transporter substrate and inhibitor may provide a preference, or selectivity, of the cells that are transduced. The vector may preferentially, or specifically, transduce some cells over others while the substrate and inhibitor may be specific, or relatively specific for ABC transporters on particular cells.

The ABC transporter substrate and inhibitors of the invention are preferably substrates that are also inhibitors of human P-glycoprotein (P-gp), but may also be a substrate inhibitor of other ABC transporters. Without binding the invention to theory, and offered to improve the understanding of the invention, the ABC transporter substrate and inhibitor may act via one or more mechanisms such as block transport of a molecule that has a stimulatory or inhibitory effect; increase transport of extracellular molecules that enhance transduction; increase or decrease export of intracellular molecules; interact with molecules associated with a viral vector; alter intracellular levels of NA⁺, CA²⁺, or K⁺; block ATPase activity of an ABC transporter; strengthen or stabilize interactions between the viral particle envelope protein, VSV-G, and the cell membrane; and strengthen or stabilize interactions between a viral membrane protein and the cell membrane. The methods of the invention may also involve one or more mechanisms other than those disclosed, but the mechanism is apparently independent of cytotoxicity and inhibition of rhodamine efflux mediated by an ABC transporter. Also, and as evident from the number and diversity of the possibilities provided, the invention cannot be limited to any one theory. Instead, and given the unusual discovery of the invention in the relationship between ABC transporter substrates and inhibitors and viral transduction efficiencies, the invention should be viewed as opening a new approach in the field of human cell therapy.

Preferred examples of ABC transporter substrates and inhibitors for use in the invention include verapamil, diltiazem, cyclosporin A, PSC833, chloroquine, and quinidine. Other non-limiting examples include the HIV protease inhibitors ritonavir, saquinavir, nelfinavir and indinavir, which have been shown to inhibit transport of some known P-gp substrates. More generically, the ABC transporter substrates and inhibitors are those that are capable of being both a substrate and an inhibitor of an ABC transporter as described herein. Of course combinations of substrates and inhibitors as described herein may also be used.

While the invention may be practiced by use of soluble forms of substrates or inhibitors, optionally crosslinked by other molecules, the substrate inhibitor compounds may optionally be immobilized on the surface of viral vector particles, such as by coating, or other solid phases, such as on beads or other solid surfaces. In one embodiment of this aspect, the compounds are immobilized on the surface of the vessel, such as the walls of a tissue culture well, plate, or bag for the viral vector mediated transduction. Without being bound by theory, use of immobilized compounds on the surface where cells adhere or make contact may increase local concentrations of compounds on the cell surface.

The invention is practiced with cells that express an ABC transporter. Preferred cells include hematopoietic stem cells derived from CD34+, CD34, SP cells, KDR+, and ABCG2+ cell populations. Particularly preferred are CD34 positive stem cell, or a differentiated progenitor cell on the dendritic cell lineage; a primary cell; or is a precursor to either of these cells. Other preferred cells for transduction in general are cells of the hematopoietic system, and in particular, hematopoietic stem cells.

In particularly preferred embodiments, the cell is a primary CD34+ hematopoietic stem cell. Preferably, the cell is of a eukaryotic, multicellular species, and, even more preferably, is of mammalian origin, e.g., a human cell. A non-limiting example is the use of the invention on CD34+ cells from Rhesus macaque with HIV based and SIV based viral vectors.

A cell to be transduced can be present as a single entity, or can be part of a larger collection of cells. Such a "larger collection of cells" include, but are not limited to, the organs/tissues/cells of the circulatory system (including for example, but not limited to heart, blood vessels, and blood) and the lymphoid system. Preferably, the cells to be transduced are those of the heart, blood vessel, including tumor blood vessels and blood vessels associated with infected or diseased tissue, bone marrow, blood, brain, lymphatic tissue, lymph node, and spleen. In one particular embodiment of the invention, the cell is autologous to the intended subject in which the cells are to be used, but cells allogenic, partially mismatched, completely mismatched, or even xenogenic to the subject may also be used. Furthermore, universal donor cells, suitable for use in any given host organism, a related group of organisms or a species, such as human beings, may be transduced. This latter embodiment of the invention is particularly important in the transplantation of cells, tissues or organs, where the source of the transduced cells may be critical to the outcome of the transplant.

Another preferred cell for transduction by the methods of the invention is a tumor cell, a diseased cell, or a cell at risk for becoming abnormal over time due to its genetic makeup or the genetic makeup of other cells present in the same organism. This is particularly relevant to such cells that have developed the expression of an ABC transporter, such as P-gp. This embodiment permits the transduced cells of the invention to be used in therapeutic treatment of tumor cells. Additional applications of the invention in cancer therapy are numerous, and one skilled in the art would be able to use the invention set out herein for the treatment of many types of cancers without undue experimentation.

The present invention includes the advantage that optionally, purification of the cell to be transduced is not essential. Transduction of mainly a cell type of interest can be accomplished by the expression of an ABC transporter in said cell type. Thus in a mixed population of blood cells, for example, transduction of cells expressing an ABC transporter, such as hematopoietic stem cells, will be enhanced when an ABC transporter substrate and/or inhibitor is used. This will occur in preference over other cell types in the population that do not express an ABC transporter.

The invention also encompasses the transduction of purified or isolated cell types if desired. The use of a purified or isolated cell type provides additional advantages such as higher efficiencies of transduction due to higher vector concentrations relative to the cell to be transduced.

The present invention includes viral vectors, and compositions comprising them, for use in the disclosed methods. Preferably, the viral vectors are derived from the family Retroviridae, which is used to include all retroviruses within the family as known to the skilled person. Non-limiting examples include avian leukosis sarcoma virus (ALSV) group (including Rous sarcoma virus, avian myelobalstosis virus, avian erythroblastosis virus, avian myelocytomatosis virus, and Rous associated virus); mammalian C-type group (including Moloney murine leukemia virus, Harvey murine sarcoma virus, Abelson murine leukemia virus, AKR-MuLV, Feline leukemia virus, Simian sarcoma virus, endogenous viruses in mammals, reticuloendotheliosis virus, and spleen necrosis virus); B-type viruses such as the mouse mammary tumor virus; D-type viruses such as Mason-Pfizer monkey virus and "SAIDS" viruses; and the HTLV-BLV group, including human T-cell leukemia (or lymphotropic) virus (HTLV)-1 and -2 as well as bovine leukemia virus. Also included are RNA viruses of the subfamily Lentiviridae, including HIV-1 and -2, simian immunodeficiency virus (SIV), feline immunodeficiency virus (FIV), bovine immunodeficiency virus, visna/maedi virus, caprine arthritis-encephalitis virus (CAEV), and equine infectious anemia virus (EIAV). Preferably, the viral vectors of the invention are a human immunodeficiency virus type 1 or 2 (i.e., HIV-1 or HIV-2, wherein HIV-1 was formerly called lymphadenopathy associated virus 3 (HTLV-III) and acquired immune deficiency syndrome (AIDS)-related virus (ARV)), or another virus related to HIV-1 or HIV-2 that has been identified and associated with AIDS or AIDS-like disease. The acronym "HIV" or terms "AIDS virus" or "human immunodeficiency virus" are used herein to refer to these HIV viruses, and HIV-related and -associated viruses, generically. Another subfamily that may be used to prepare viral vectors of the invention is Spumaviridae, which includes simian foamy virus, human foamy virus, human spumareetrovirus, feline syncytium-forming virus.

A particularly preferred viral vector is one derived from HIV, most preferably HIV-1, HIV-2, or chimeric combinations thereof. Of course different serotypes of retroviruses, especially HIV, may be used singly or in any combination to prepare vectors for use in the present invention. Preferred vector nucleic acids of the invention include a "basic" lentiviral vector nucleic acid, containing minimally, LTRs and packaging sequences in the 5' leader and gag encoding sequences. Such a vector nucleic acid can also optionally contain the RRE element to facilitate nuclear export of vector RNA in a Rev dependent manner. The vector nucleic acid may also contain cis acting elements that are present in the wild-type virus, but not present in a basic vector.

A preferred vector nucleic acid contains a central polypurine tract (cppt) and central termination sequence (cts) that is capable of expressing a therapeutic antisense sequence complementary to a wildtype viral sequence. Other examples of vectors for use in the present invention are described in US patent 5,885,806. The constructs in patent 5, 885,806 are merely examples that do not limit the scope of vectors that may be used in combination with the present invention. Instead, the constructs provide additional guidance to the skilled artisan that a viral vector for use with the present invention may contain minimal sequences from the wild-type virus or contain sequences up to almost the entire genome of a wild-type virus.

Furthermore, placing sequences from other viral backbones into viral vectors of interest is also well known in the art. Regardless of the actual viral vector used, various accessory proteins encoded by, and sequences present in, the viral genetic material may be left in the vector if these proteins or sequences increase transduction efficiency in cells. Numerous routine screens are available to determine whether certain genetic material increases transduction efficiency by incorporating the sequence in either the vector or helper genomes. A preferred embodiment of the invention is to not include accessory proteins in the vector genome. But this preference does not exclude embodiments of the invention where accessory proteins and other sequences are left in either the vector to further increase transduction efficiency.

The viral vectors used in the present invention result from "pseudotype" formation, where co-infection of a cell by different viruses produces progeny virions containing the genome of one virus encapsulated within an outer layer containing one or more envelope protein of another virus. This phenomenon has been used to package lentiviral vectors of interest in a "pseudotyped" virion by co-transfecting or co-infecting a packaging cell with both the lentiviral vector of interest and genetic material encoding at least one envelope protein of another virus or a cell surface molecule. See U.S. Patent 5,512,421. Such mixed viruses can be neutralized by anti-sera against the one or more heterologous envelope proteins used. One virus commonly used in pseudotype formation is the vesicular stomatitis virus (VSV), which is a rhabdovirus. The use of pseudotyping broadens the host cell range of the virus by including elements of the viral entry mechanism of the heterologous virus used.

Pseudotyping of viral vectors with VSV for use in the present invention results in viral particles containing the viral vector nucleic acid encapsulated in a-nucleocapsid which is surrounded by a membrane containing the VSV G protein. The nucleocapsid preferably contains proteins normally associated with the viral vector. The surrounding VSV G protein containing membrane forms part of the viral particle upon its egress from the cell used to package the viral vector. Examples of packaging cells are described in U.S. Patent 5,739,018. In a preferred embodiment of the invention, the viral particle is derived from HIV and pseudotyped with VSV G protein. Pseudotyped viral particles containing the VSV G protein can infect a diverse array of cell types with higher efficiency than amphotropic viral vectors. The range of host cells include both mammalian and non-mammalian species, such as humans, rodents, fish, amphibians and insects. Other proteins that may be used to pseudotype viral vectors of the invention include envelope proteins of HIV-1 or HIV-2; the MMLV envelope protein; the G protein from Vesicular Stomatitis Virus (VSV), Mokola virus, or rabies virus; GaLV, Alphavirus El/2 glycoprotein; or RD114, an env protein from feline endogenous virus.

The viral vector for use in the transduction methods of the invention can also comprise and express one or more nucleic acid sequences under the control of a promoter present in the vector nucleic acid or under the control of an introduced, heterologous promoter. The promoters may further contain insulatory elements, such as erythroid DNAse hypersensitive sites, so as to flank the operon for tightly controlled gene expression. Preferred promoters include the HIV-LTR, CMV promoter, PGK, Ul, EBER transcriptional units from Epstein Barr Virus, tRNA, U6 and U7. While Pol 11 promoters are preferred, Pol III promoters may also be used. Tissue specific promoters are also preferred embodiments. For example, the beta globin Locus Control Region enhancer and the alpha & beta globin promoters can provide tissue specific expression in erythrocytes and erythroid cells. Another further preferred embodiment is to use cis-acting sequences that are associated with the promoters. For example, The U 1 gene may be used to enhance antisense gene expression where non-promoter sequences are used to target the antisense or ribozymes molecule to a target spliced RNA as set out in US patent 5,814,500.

Of course any cis acting nucleotide sequences from a virus may be incorporated into the viral vectors of the invention. In particular, cis acting sequences found in retroviral genomes are preferred. For example, cis-acting nucleotide sequence derived from the gag, pol, env, vif, vpr, vpu, tat or rev genes may be incorporated into the viral vectors of the invention to further increase transduction efficiency. Preferably, a cis acting sequence does not encode an expressed polypeptide; is not expressed as a polypeptide or part thereof due to genetic alteration, such as deletion of a translational start site; encodes only a portion or fragment of a larger polypeptide; or is a mutant sequence containing one or more substitutions, additions, or deletions from the native sequence. An example of a cis acting sequence is the cPPT (central polypurine tract) sequence identified within the HIV pol gene.

Another sequence for possible inclusion is one previously identified sequence that is insufficient to significantly increase transduction efficiency, and described by Zennou et al. (2000) as a central DNA flap (a 178 base pair fragment from positions 4793 to 4971 on pLAI3, corresponding to positions 4757 to 4935 on pNL4-3) capable of increasing transduction efficiency. The present invention may also take advantage of the discovery that while this small fragment is not sufficient to increase the transduction efficiency, a larger 545 base pair fragment (positions 4551 to 5096 in pNL4-3), or yet larger fragments containing it, is capable of increasing transduction.

Said one or more nucleic acid sequences in the iviral vector nucleic acids of the invention may be found in the virus from which the vector is derived or be a heterologous sequence. The sequence is preferably a full-length or partial sequence that is or encodes a gene product of interest. Such sequences and gene products are preferably biologically active agents capable of producing a biological effect in a cell. Examples of such agents include proteins, ribonucleic acids, enzymes, transporters or other biologically active molecules.

In one preferred embodiment, the agent is a protein, such as a toxin, transcription factor, growth factor or cytokine, structural protein, or a cell surface molecule. The protein may contain one or more domains for which no function has been identified and may be homologous to the transduced cell. Additionally, the protein may be absent, deficient or altered in the cell to be transduced. Alternatively, the protein may be a transdominant negative mutant or a decoy to prevent a natural protein from carrying out its normal activity in the transduced cell.

For example, the nucleic acid sequence may code for a ribozyme that binds, cleaves and destroys RNA expressed, or to be expressed, in the transduced cell. Alternatively, the nucleic acid sequence may code for an antisense molecule designed to target a particular nucleic acid sequence and result in its degradation. The vector contained sequence may be overexpressed, inducibly expressed, or under cellular or viral regulatory transcription control in the transduced cell. Depending on the intended use, the heterologous sequence may encode any desired protein including a marker for transduced cells. Such markers include selectable markers such as a particular resistance phenotype, such as neomycin, MDR-1 (P-glycoprotein), O6-methylguanine-DNA-methyltransferase (MGMT), dihydrofolate reductase (DHFR), aldehyde dehydrogenase (ALDH), glutathione-S-transferase (GST), superoxide dismutase (SOD) and cytosine deaminase.

In the methods of the invention, the cells to be transduced are exposed to contact with the ABC transporter substrate and inhibitor before, or simultaneously with, application of the viral vector. For example, the cells can be cultured in media with the substrate and/or inhibitor before contact with, or in the presence of, the viral vector to be transduced. Preferably, the cells are exposed to the substrate and inhibitor before and during contact with the viral vector. Under these conditions, the substrate and inhibitor is not washed away from the cells prior to actual transduction with the viral vector.

Incubation of the cells with the viral vector may be for different lengths of time, depending on the conditions and materials used. Factors that influence the incubation time include the cell, vector and MOI (multiplicity of infection) used, the inhibitor(s) and amounts used, whether and how said molecule(s) are immobilized or solubilized, and the level of transduction efficiency desired. Preferably, the incubation is for about eight to about 72 hours, more preferably for about 12 to about 48 hours. In a particularly preferred embodiment, the incubation is for about 24 hours and is optionally repeated once.

Contact between the cells to be transduced and a viral vector occurs at least once, but it may occur more than once, depending upon the cell type. A preferred method of the invention is to introduce a viral vector into the medium of cells that already contains an ABC transporter substrate and/or inhibitor to avoid changing the medium. Transduction can proceed for as long as the conditions permit without the process being significantly detrimental to the cells or the organism containing them.

Similarly, the MOI used is from about I to about 400, preferably less than 500. Generally, the preferred MOI is from about 2 to about 100. More preferably, the MOI is from about 10 to about 50, although ranges of from about 1 to about 10, about 20, about 30, or about 40 are also contemplated. Most preferred is an MOI of about 20 or 25. Furthermore, the copy number of viral vector per cell should be at least one. However, manycopies of the vector per cell may also be used with the above described methods. The preferred range of copies per cell is from about 1 to about 100. The more preferred copy number is the minimum copy number that provides a therapeutic, prophylactic or biological impact resulting from vector transduction or the most efficient transduction.

For therapeutic or prophylactic applications, a more preferred copy number is the maximum copy number that is tolerated by the cell without being significantly detrimental to the cell. Both the minimum and maximum copy number per cell, as well as the amount of ABC transporter substrate and inhibitor, will vary depending upon the cell to be transduced as well as other cells that may be present. The optimum copy number and substrate and inhibitor concentration is readily determined by those skilled in the art using routine methods. For example, cells are transduced at increasing increments of multiplicities of infection and/or concentration of substrate and inhibitor. The cells are then analyzed for copy number, therapeutic or biological impact and for detrimental effects on the transduced cells or a host containing them (e.g. safety and toxicity). One non-limiting example is to examine cell survival after transduction.

After incubation with the viral vector *in vitro,* the cells may be cultured in media for various times before the cells are analyzed for the efficiency of transduction or otherwise used. Such culturing may be under any conditions that result in cell growth and proliferation, such as incubation with interleukin-2 (IL-2) or with a cell surface binding molecule (such as, but not limited to, CD3 and/or CD28 antibodies or a FLT-3 ligand, TPO or Kit ligand). Post transduction incubation may be for any period of time, but is preferably from about one to about seven to ten days. Longer periods of time, such as about 14 days, may also be used, although periods that are detrimental to cell growth are not preferred. In preferred embodiments of the invention, the cells are cultured for about 24, about 48, about 72, or about 96 hours followed by a wash in vector-free and inhibitor-free media that removes excess vector and inhibitor. The cells are then optionally cultured with or without viral vector for times ranging from about 24 to about 72 hours, or used without further culturing.

The efficiency of transduction observed with the practice of the present invention (and in the presence of a substrate and inhibitor) is from about 30 to about 100%. Preferably, the efficiency is at least about 40 to 80%. More preferred embodiments of the invention are where transduction efficiency is between about 30 and about 60%. The increase in efficiency of transduction can also be expressed as a relative increase over the level of efficiency in the absence of a substrate or inhibitor. This relative increase is from about two-fold to about six-fold or more over the efficiency of transduction in the absence of a substrate and inhibitor. This increase would result in a transduction efficiency of preferably about 50% to about 100%.

The transduced cells may be used in research or for treatment or prevention of disease conditions in living subjects. Therapeutic uses for the transduced cells include the introduction of the cells into a living organism. For example, unstimulated primary T cells isolated from an individual infected with, or at risk of being infected with HIV, may be first transduced by a vector, like that described in US Patent 5,885,806, using the present methods and followed by injection of the transduced cells back into the individual. Alternatively, the cells may be used directly for the expression of a heterologous sequence present in the lentiviral vector. The protein expressed by the sequence may be used in research or treatment.

When used as a part of HIV therapy or prophylaxis, the viral vector may encode a toxin or other anti-viral agent that has been adapted for anti-HIV applications. Alternatively, the vector may encode an agent designed to target HIV, such as transdominant negative mutants of the tat, rev, nef, vpu, or vpr genes. In other applications the transduced cell may be corrected to express an appropriate globin gene to correct sickle cell anemia or thalassaemia. Immune cells may also be transduced to modulate their immune function, their response to antigen, or their interactions with other cells. The skilled artisan is aware of the above uses for the present transduction methods as well as numerous other uses and applications known in the art.

In a further aspect of the invention, methods of determining the level of increased transduction of retroviral and lentiviral vectors in cells, mediated by a known or putative ABC transporter substrate and inhibitor, are provided. In one embodiment, these methods comprise contacting a cell expressing one or more ABC transporter with a known or putative substrate or inhibitor compound and a viral vector as described herein. The transduction efficiency in the presence of such a compound may be compared to that in the absence of the compound to identify the level of increased transduction of the vector into the cell. These methods may be practiced repeatedly, with a variety of amounts or concentrations of the compound to determine the level of increased transduction over a range of conditions. The methods may also be used to determine that the level of increase is undetectable.

An exemplary method of determining the level of increased transduction of a retroviral vector in a cell mediated by an ABC transporter substrate or inhibitor compound may comprise
a) providing a population of said cell;
b) contacting one or more cells of said population with said vector in the presence of said compound to produce a first contacted cell or cells and contacting a second cell or cells of said population with said vector in the absence of said compound to produce a second contacted cell or cells; and
c) determining the level of transduction with said vector in said first contacted cell or cells and in said second contacted cell or cells.

If the levels of transduction are identical, or nearly so, then the level of increase is zero or nearly so. If a higher level of transduction is seen in said second contacted cell or cells, then the assayed compound increases transduction efficiency of said vector in said population of cells. The population of cells are preferably primary cells, optionally from a human being and optionally already known or suspected to express an ABC transporter inhibitor.

The determination of the level of transduction can be made in a variety of ways as would be known to the skilled practitioner. Non-limiting examples include detection of the presence and/or amount of genetic material encoded by the vector in said cells, such as by PCR, expression of nucleic acid sequence(s) (e.g. real time PCR or protein detection), which can also be conducted *in vivo* by injecting a compound into animal in combination with administration of a vector followed by detection of transduction efficiency as described above; and a phenotypic change in the cell mediated by the vector, such drug resistance, reporter gene expression, cellular toxicity, restoration of a deficient pathway. Other examples include using a replication competent virus to determine replication rate in the presence or absence of compounds where compounds that increase transduction would promote virus replication (optionally performed competitively with more than one virus in culture or with a single virus); using *ex vivo* hematopoietic assays such as CFU (colony forming units), LTC-IC (long term culture-initiating cell), and ML-IC (myeloid-lymphoid-initiating cell) to detect cells that have been transduced; using *in vivo* hematopoietic assays such as xenograft transplants such as NOD-SCID and SCID-hu, syngeneic transplant, autologous transplant or allogeneic transplant to detect cells that have been transduced; measuring transduction levels into cell lines that overexpress one or more specific ABC transporters compared to cell lines that lack expression of the transporter(s); and measuring transduction into cells that express ABC transporters compared to cells with low to no ABC transporter activity.

Other means to determine the level of transduction include those based on effects of a compound on ABC transporters expressed by the cells. Non-limiting examples include ABC transporter activity assays or inhibition assays, such as Rhodamine efflux or Hoescht flow cytometry; ABC transporter substrate transport assays, such as using a fluorescent dye or labeled molecule to measure transport of a substrate through an ABC transporter; ATPase activity assays or inhibition assays; ionic transport activity assays or inhibition assays; Hoescht stain and flow cytometry to measure changes in the percentage of SP cells in a population of primary cells; measuring cytotoxicity in the presence or absence of compounds; labeling liposomes with a substrate and/or inhibitor compound and determining liposomal uptake; absorption flux assays, including polarized transport assays; and detecting cellular accumulation of a substrate and/or inhibitor compound.

Less direct means to determine the level of transduction may also be used. These include, but are not limited to, (high throughput) phage display of ABC transporters to detect interactions with a substrate and/or inhibitor compound; (high throughput) phage display of a substrate and/or inhibitor compound to detect interactions with an ABC transporter; and measuring metabolism rate of a substrate and/or inhibitor compound (for example CYP3A metabolism of verapamil).

### Brief Description of the Drawings

The patent or application file contains at least one drawing executed in color. Copies of this patent or patent application publication with color drawing(s) will be provided by the Office upon request and payment of the necessary fee.

Figure 1, panels A and B show transduction efficiencies of clinical grade vectors in the presence and absence of verapamil.

Figure 2, panels A and B show transduction efficiencies of research grade vectors in the presence and absence of verapamil.

Figure 3, panels A and B show transduction efficiencies of clinical grade vectors in the presence and absence of an HIV protease inhibitor.

Figure 4 shows transduction levels in total human hematopoietic cells (CD45+), myeloid (CD33+), lymphoid (CD19+) and primitive hematopoietic cells (CD34+) obtained from 6-8 week old NOD-SCID mice transplanted with transduced human cord blood CD34+ cells.

Figure 5 shows transduction levels in total human hematopoietic cells (CD45+), myeloid (CD33+), lymphoid (CD19+) and primitive hematopoietic cells (CD34+) obtained from 14 week old NOD-SCID mice transplanted with transduced human cord blood CD34+ cells.

### Modes of Carrying Out the Invention

The present invention is based in part on the surprising discovery that transduction in the presence of verapamil, an ABC transporter inhibitor, dramatically enhances transduction efficiency into total CD34+ cells from the bone marrow, G-CSF mobilized peripheral blood, and cord blood, as well as progeny colony forming units (CFU), long term culture-initiating cells (LTC-IC) and SRC engrafted into NOD-SCID mice. Verapamil increases of gene transfer into CD34+ cells when transduced with clinical, or research, grade lentiviral vector.

A cppt containing lentiviral vector manufactured at a small scale for research purposes transduced human mobilized peripheral blood and cord blood derived CD34 positive cells at up to 70% efficiency, and SCID repopulating cells (SRC) at up to 97% efficiency in the absence of an ABC transporter substrate inhibitor. Clinical grade vectors produced by large scale manufacturing, including stringent purification of vector and safety procedures that reduce the likelihood of recombinant replication competent lentivirus, however, do not have such high levels of transduction. The transduction efficiency of the clinical grade vector into CD34 positive cells in the absence of an ABC transporter substrate inhibitor is on the order of 10% to 30%.

Surprisingly, the addition of verapamil during culture restored the transduction capacity of large-scale clinical grade vector, transducing human CD34 positive cells from mobilized peripheral blood, bone marrow and cord blood at efficiencies between 50% and 80%. This represents a two- to six-fold increase in transduction efficiency above clinical grade vector transduction levels in the absence of verapamil. The increase was consistently observed among different sources of CD34 positive cells. Exposure to verapamil did not reduce CD34 expression levels. The vector copy number and gene expression were stable over 5-week (long term culture (LTC), and total CD34 and secondary CFU derived from LTC-IC were transduced comparably. Human SRC recovered from the bone marrow of NOD-SCID mice were transduced at 80% to 90% efficiency, representing a three to four-fold increase over transduction levels achieved in the absence of verapamil.

The frequency and distribution of hematopoietic subsets in NOD-SCID mice were also unchanged when transduced in verapamil, confirming that verapamil did not alter the multipotentiality of the cultured CD34 cells. Multilineage transduction, including T cells in the thymus, was observed. Efficient hematopoietic stem cell transduction was observed, as transduction levels remained stable in primary and secondary transplanted mice.

In addition to verapamil, diverse pharmacological agents that commonly interfere with ABC transporters were found to enhance transduction. Preferred for use with the present invention are inhibitors of P-glycoprotein, while inhibitors of MRP may also be used. Diltiazem and quinidine increased transduction to levels similar to verapamil, while probenecid, an MRPI and MRP2 inhibitor, increased transduction in hematopoietic cells less than 2-fold. Moreover, post-transduction exposure to verapamil did not increase expression levels from the vector promoter, therefore, and without being limited by theory, transduction enhancement does not appear to be due to an increase in vector expression.

The present invention is directed to methods, and compositions related thereto, for the transduction of cells with lentiviral vectors with use of an ABC transporter substrate and inhibitor. The transduced cells may be distinguished from non-transduced cells by any appropriate means known in the art, including, but not limited to, the expression of a reporter or marker molecule, such as a green fluorescent protein (GFP) or a selectable marker, or the detection of transduced nucleic acids, such as by PCR or other nucleic acid detection methods. The methods relate to the fact that contact of the cells to be transduced with at least one ABC transporter substrate and inhibitor increases the efficiency of transduction. The invention is advantageously applied to improve any application of gene therapy by increasing transduction efficiency but may also be applied to applications of viral vectors for use in protein production and manufacturing and for use in propagation of viruses.

Cells for use with the present methods are preferably unstimulated primary cells, which are freshly isolated, or frozen, from an *in vivo* source, optionally cultured for various times in media that may include the presence of factors which maintain them in a proliferating state. A non-limiting example includes CD34- cells that contain hsc. Primary cells from an *in vivo* source may be optionally selected for particular cell types. For example, if primary CD34+ cells are to be used, peripheral blood (PB) or cord blood ("CB" from an umbilical source) or bone marrow samples are first obtained followed by enrichment for CD34+ cell types. Standard magnetic beads positive selection, plastic adherence negative selection, and/or other art recognized standard techniques may be used to isolate CD34+ cells away from contaminating PB cells. Purity of the isolated cell types may be determined by immunophenotyping and flow cytometry using standard techniques.

After isolation, the primary cells may be used in the methods of the invention to be transduced with viral vectors at increased efficiencies because of the presence of ABC transporter substrates and/or inhibitors. The invention is most advantageously used with primary hematopoietic stem cells (hsc), such as those in KDR+, ABCG2+ and SP cell populations, transduced with an HIV-1 based vector capable of expressing heterologous genetic material of interest. Another preferred use is with the CD34 positive cells of any *in vivo* blood source. In cases where the heterologous genetic material is or encodes a therapeutic or prophylactic product for use *in vivo* to treat or prevent a disease, the transduced primary cell can be introduced back into an *in vivo* environment, such as a patient. As such, the invention contemplates the use of the transfected cells in gene therapy to treat, or prevent, a disease by combating a genetic defect or targeting a viral infection.

For the transduction of primary cells in a mixed population, the above isolation/purification steps would not be used. Instead, the cell to be transduced would be selection by virtue of expression of an ABC transporter that interacts with the substrate and/or inhibitor used. For example, CD34+ cells can either be first purified and then transduced by the methods of the invention or alternatively be transduced as part of a mixed population, like cord blood cells or peripheral blood cells by use of the same methods. Hematopoietic stem cells in total white blood cell populations, which may be difficult to purify or isolate, may be transduced in the mixed populations by use of the present invention.

The present invention includes compositions comprising a substrate and inhibitor and a viral vector for use as part of the disclosed methods. An exemplary composition comprises the substrate and inhibitor compound and a viral vector to be transduced, optionally in the presence of the cells to be transduced. Another composition is the substrate and inhibitor compound and the cells to be used in a kit form ready for the transduction of a viral vector of choice. The vectors are lentiviral vectors. A particularly preferred lentiviral vector is one derived from a Human Immunodeficiency Virus (HIV), most preferably HIV-1, HIV-2, or chimeric combinations thereof. Of course different lentiviral vectors may be simultaneously transduced into the same cell by use of the present methods. For example, one vector can be a replication deficient or conditionally replicating lentiviral vector while a second vector can be a packaging construct that permits the first vector to be replicated/packaged and propagated. When various viral accessory proteins are to be encoded by a vector, they may be present in any one of the vectors being transduced into the cell. Alternatively, the viral accessory proteins may be present in the transduction process via their presence in the viral particles used for transduction. Such viral particles may have an effective amount of the accessory proteins co-packaged to permit the practice of the present invention. In a preferred embodiment, the viral vector does not encode one or more of the accessory proteins.

A lentiviral vector for use in the transduction methods of the invention can also comprise and express one or more nucleic acid sequences under the control of a promoter. In one embodiment of the invention, a nucleic acid sequence encodes a gene product that, upon expression, would alleviate or correct a genetic deficiency in the cell to be transduced. In another embodiment, the nucleic acid sequence encodes or constitutes a genetic antiviral agent that can prevent or treat viral infection. By "genetic antiviral agent", it is meant any substance that is encoded or constituted by genetic material. Examples of such agents are provided in U.S. Patent 5,885,806. They include agents that function by inhibiting viral proteins, such as reverse transcriptase or proteases; competing with viral factors for binding or target sites; or targeting viral targets directly for degradation, such as in the case of ribozymes and antisense constructs. Other examples of genetic antiviral agents include antisense, RNA decoys, transdominant mutants, interferons, toxins, nucleic acids that modulate or modify RNA splicing, immunogens, and ribozymes, such as "hammerhead" and external guide sequence (EGS) mediated forms thereof.

As noted above, a lentiviral vector can encode a marker or reporter for transduced cells. In the examples presented in the figures and below, green fluorescent protein (GFP) is the marker encoded by the viral vector transduced into CD34+ cells. Other markers include those described above. Detection of GFP may serve to identify the number of functionally transduced cells, which were not only transduced with the vector, but were also able to functionally express GFP to levels that could be detected by FACS analysis. It should be noted that the detection may not represent the actual number of transduced cells since some cells may have been transduced with the vector but express GFP at levels that are below the limits used in FACS detection.

An alternative approach to detecting transfection efficiency is with the polymerase chain reaction (PCR). For example, TaqMan PCR can be used to determine the actual number of copies of integrated lentiviral vector in a transduced cell.

The cells to be transduced may be exposed to contact with the substrate and inhibitor either before, or simultaneously with, contact with the viral vector. Thus the cells can be first exposed to the inhibitor for a period of time followed by introduction of the vector. Such cells may be newly isolated or prepared primary cells that have not been intentionally stimulated to enter the cell cycle or that have been cultured in the presence of one or more cytokines. After contact with the vector, excess vector is preferably not removed and the cells cultured under conditions conducive to cell growth and/or proliferation. Such conditions may be in the presence of the cell surface binding molecule or other stimulatory/activating factors, such as cytokines and lymphokines in the case of cells responsive thereto. Alternatively, excess vector may be removed after contact with the cell and before further culturing.

Another embodiment of the invention is to culture the cells in the presence of both viral vector and substrate and inhibitor molecule simultaneously. Such cells are may optionally be previously stimulated or concurrently stimulated with cytokines or other stimulating factors. After a period of time, the cells are washed of excess vector and substrate and/or inhibitor and cultured under growth or proliferation inducing conditions such as the presence of stimulatory/activating factors.

In any of the above combinations of viral vector and cell surface binding molecule administration, incubation with the vector can be optionally repeated at least once. Contact with the vector can also be repeated more than once, such as twice, thrice, four times, or more.

The length of incubation of viral vector and the cells to be transformed is preferably for about 24 hours. Preferably, incubation with a substrate and inhibitor molecule occurs simultaneously with contact of the cells with the viral vector. Under such circumstances, the substrate and inhibitor molecule may be left in contact with the cells when the vector is introduced. Following incubation, the cells are preferably kept in culture in the presence of vector but not substrate or inhibitor.

After contact with the vector, the cells are cultured under conditions conducive to their growth or proliferation. An example is a factor conducive to cell growth, such as interleukin-2. Other factors include mitogens such as phytohemaglutinin (PHA) and cytokines, growth factors, activators, cell surface receptors, cell surface molecules, soluble factors, or combinations thereof, as well as active fragments of such molecules, alone or in combination with another protein or factor, or combinations thereof. Non-limiting examples of additional factors include epidermal growth factor (EGF), transforming growth factor alpha (TGF-alpha), angiotensin, transforming growth factor beta (TGF-beta), GDF, bone morphogenic protein (BMP), fibroblast growth factor (FGF acidic and basic), vascular endothelial growth factor (VEGF), PIGF, human growth hormone (HGH), bovine growth hormone (BGH), heregulins, amphiregulin, Ach receptor inducing activity (ARIA), RANTES (regulated on activation, normal T expressed and secreted), angiogenins, hepatocyte growth factor, tumor necrosis factor beta (TNF-beta), tumor necrosis factor alpha (TNF-alpha), angiopoietins 1 or 2, insulin, insulin growth factors I or II (IGF-I or IGF-2), ephrins, leptins, interleukins 1, 2, 3, 4, 5, 6, 7, 8, 9, 10, 11, 12, 13, 14, or 15 (IL-1, IL-2, IL-3, IL-4, IL-5, IL-6, IL-7, IL-8, IL-9, IL-10, IL-11, IL-12, IL-13, IL-14, or IL-15), G-CSF (granulocyte colony stimulating factor), GM-CSF (granulocyte-macrophage colony stimulating factor), M-CSF (macrophage colony stimulating factor), LIF (leukemia inhibitory factor), angiostatin, oncostatin, erythropoietin (EPO), interferon alpha (including subtypes), interferons beta, gamma, and omega, chemokines, macrophage inflammatory protein-1 alpha or beta (MIP-1 alpha or beta), monocyte chemotactic protein-1or-2 (MCP- or 2), GRO beta, MIF (macrophage migration inhibitory factor), MGSA (melanoma growth stimulatory activity), alpha inhibin HGF, PD-ECGF, bFGF, lymphotoxin, Mullerian inhibiting substance, FAS ligand, osteogenic protein, pleiotrophin/midkine, ciliary neurotrophic factor, androgen induced growth factor, autocrine motility factor, hedgehog protein, estrogen, progesterone, androgen, glucocorticoid receptor, RAR/RXR, thyroid receptor, TRAP/CD40, EDF (erythroid differentiating factor), Fic (growth factor inducible chemokine), IL-1RA, SDF, NGR or RGD ligand, NGF, thymosine-alphal, OSM, chemokine receptors, stem cell factor (SCF), or combinations thereof. As evident to one skilled in the art, the choice of culture conditions will depend on knowledge in the art concerning the cells transduced as well as the subsequent intended use of the cells. For example, the combination of IL-3,1L-6 and stem cell factor is used in some clinical trials. Similarly, the choice of culture conditions would preferably not be to the detriment of cell viability or transduction efficiency.

Preferably, the post transduction incubation is for a period of about four hours, or for about one to about seven to ten days. More preferably from about 16 to about 20 hours or for about four, about five or about six days. About fourteen days of post-transduction incubation is also contemplated. The length of incubation depends upon the cells used and the application desired. Tranduced cells may be washed immediately after incubation and then frozen or transplanted into a subject. This is particularly preferred for cells and applications that do not benefit from expansion in culture, such as hematopoietic stem cells. Alternatively, cells to be use for protein production via expression of a viral vector borne coding sequence may be cultured for longer periods after incubation.

In addition to the above, the transduced cells may be used in research or for treatment of disease conditions in living subjects. Particularly preferred as part of the invention are therapeutic uses for the transduced cells to produce gene products of interest or for direct introduction into a living organism as part of gene therapy. For example, and as exemplified below, primary hsc cells can be transduced with a lentiviral vector. Successful transduction is indicated by the production or overproduction of a gene product encoded by the vector or generation of a phenotype conferred by the vector. As such, primary hsc cells can be first transduced with a vector containing, and capable of expressing, desirable or useful nucleic acid sequences, and then returned to an *in vivo* environment such as a living subject. Preferably, the living subject is an individual infected with, or at risk of being infected with HIV-1.

In another embodiment, the hsc cells are transduced with genes or nucleic acids capable of conditionally killing the hsc cells upon introduction into a host organism. This has applications in allogenic bone marrow transplantation to prevent graft versus host disease by killing hsc cells with a pro-drug approach.

Alternatively, the primary cells can be deficient in a gene product, and the deficiency correctable by the transduced viral vector. Such cells would be reintroduced into the living subject after transduction with the vector.

Thus, both *in vitro* and *ex vivo* applications of the invention are contemplated. For transfers into a living subject, the transduced cells are preferably in a biologically acceptable solution or pharmaceutically acceptable formulation. Such a transfer may be made intravenously, intraperitoneally or by other injection and non-injection methods known in the art. The dosages to be administered will vary depending on a variety of factors, but may be readily determined by the skilled practitioner. There are numerous applications of the present invention, with known or well designed payloads encoded or borne by a viral vector, where the benefits conferred by the transduced genetic material will outweigh any risk of negative effects.

Initially, the total number of transduced cells transferred would be from about 10^{a} to about 10¹⁰. As such, 10⁵, 10⁶, 10⁷, 10⁸, or 10⁹ cells may be used. The actual numbers will vary depending on the cells being transduced. As a non-limiting example, and for hsc transplants, the number of CD34 cells transplanted are determined by patient body weight, such as from about 2 x 10⁶ CD34 cells/kg to about 5 x 10⁶ CD34 cells/kg although intermediate numbers such as about 3 x 10⁶ or about 4 x 10⁶ may also be used. Multiple transfers, if required, of transduced cells are a preferable embodiment. Furthermore, conditioning of the host prior to the transfer of transduced cells, if required, is a preferable embodiment. Conditioning regimens are known in the art; an example is the regimen(s) for bone marrow transplantation.

### ABC transporter substrate and/or inhibitors

Most ABC transporter inhibitors are also substrates of the transporter, and the invention is readily practiced by using a substrate and inhibitor with a cell that expresses the cognate transporter for said substrate and inhibitor. Non-limiting examples of substrates and inhibitors for use in the invention include verapamil, diltiazem, quinindine, ritonavir, probenecid, vitamin E (Tocopherol), vinca alkaloids, anthracyclines, epipodophyllotoxins, and vincristine. In some embodiments of the invention, vitamin E is preferably not used.

Other substrates/inhibitors include Amiloride; Amiodarone; Amytriptyline (as well as its increased effect when co-administered with TNF-α); Atorvastatin; Aureobasidin and analogues; B669; B-859-35 (R-enantiomer) and its major metabolite; Berrylium fluoride (BeFx); Calmodulin inhibitors; Chloroquine; Chloropromazine; Clofazimine; Cremophor EL; Cyclosporin A; FK 506; rapamycin; ascomycin; Daunorubicine; daunomycine; morpholine; doxorubicine; ; Dipyridamole; Erythromycine; Fluoroquinolones (fleroxacin, enoxacin, grepafloxacin, levofloxacin, norfloxacine); Glibenclamides and analogues; Gluconate salts; Gramicidin; Hydrocortisone; Itraconazole; Lidocaine; MS-209 (quinolone derivative); PAK-104p; Phosphatidyl-choline; Pristinamycin la; Propafenone, Amiodarone; Propranolol (racemic); Pyridine analogue; Quercetin 4'-b-glucoside; Quinine; quinacrine; cinchonine; SDZ PSC 833; SDZ 280-446; synthetic isoprenoids; Talinolol; Tamoxifen and metabolites; Taxoid (Tetracyclic taxopine C and derivatives); Terfenadine; TMP-substituted phenazines; Triton X100 (detergent); Valinomycin; nifedipine; bepridil; nicardipine; niguldipine (S-enantiomer); nitrendipine; trifluoperazine; felodipine; Vinblastine; and Vindesine.

Other non-inhibitor substrates include Actinomycin D; Adriamycin; Aldosterone; β-Lactam antibiotics celiprolol, DMP 728, and Adefovir; Benzo[a]pyrene; C6 NBD-glycosylceramide (short chain fatty acid); C6 NBD-phosphatidyl choline (short chain fatty acid); Calcein AM; Colchicine; Cyclic & linear Peptides (N-Acetyl-leucyl-norleucine, Valinomycin, Yeast α-factor pheromone); Dexamethasone; Cortisol; Hydrocortisone; Digoxin; Emetine; Estradiol; Ethidium bromide; Etoposide; Teniposide; 7,12-dimethylbenz[a]anthracene; Fexofenadine; Fluo-3 (Calcium indicator); Hoechst 33342; L-DOPA; Losartan; Metkephamid; Mithramycin; Mitomycin C; Morphine; Paclitaxel; Docetaxel; Phosphatidylcholine; Phosphatidylethanolamine; Propantheline; Puromycin; Rhodamine 123; Tc99m sestamibi; and Topotecan.

Substrates and inhibitors may be classified based on the subfamilies of ABC transporters that use or are inhibited by the substrates and inhibitors. The ABC transporter family has been divided into seven subfamilies referred to as ABCA (also known as ABC1), ABCB (also known as MDRlTAP), ABCC (also known as CFTR/MRP), ABCD (also known as ALD), ABCE (also known as OABP), ABCF (also known as GCN20), and ABCG (also known as white). Substrates and inhibitors of each of these subfamilies may also be used in the practice of the invention. Exemplary substrates of MRP1 that may be used in the practice of the present invention include Actinomycine; C6 NBD-glycosylceramide (short chain fatty acid); Calcein AM; Daunorubicin; Dinitrophenol; Glucuronid of Bilirubin, Doxorubicin, Estradiol, Hyodeoxycholate, and VP16; Glutathione conjugate of Aflatoxin B1, C6-NBD-Phosphatidylserine, Chlorambucil, Daunomycin (WP814), Daunorubicin (WP811 & WP813), Dinitrophenol (DNP-SG), Doxorubicin, Prostaglandin Al, LTC4, and Maleic acid; Digoxin; Fluo 3; Methotrexate; N-ethylmaleimide-S-glutathione; Oxidized glutathione; and Para-aminohippurate. Exemplary inhibitors include Arsenate; Benzbromarone; Cyclosporin A; Dipyridamole; Furosemide; Gamma-GS(naphtyl)cysteinyl-glycine diethyl ester; Genistein; Glutathione; Indomethacin (high concentration); LTC4; LTD4 receptor antagonist; 2K112993 (steroides); MK571; MS209 (quinolone derivative); Azidophenylacyl; PAK-104p; Penicillin G; Probenecid; Pyridine analogue; Quinidine; Rifampicin; RU 486; SDZ-PSC 833; SDZ 280-446; Sodium vanadate; Sulfinpyrazone; Verapamil; and Vinblastine.

Exemplary substrates of MRP2 that may be used in the practice of the present invention include BCECF (2', 7'-bis(2-carboxyethyl)-5(6)-carboxyfluorescein); Bilirubin-diglucuronid (and other phase 2 biotransformation products); BQ123 (cyclic pentapeptide, endothelin receptor antagonist); Calcein AM; Chrysin; Digoxin; DNP-SG (2,4-dinitrophenyl S-glutathione); Endothelin I; Estradiol 17b-D-glucuronide; Genistin; Glutathione-S-S-glutathione; Glutathion-methylfluorescein; Grepafloxacin; Nethylmaleimide-S-glutathione; PAH; Penicillin G; Pravastatin; SN 38 (CPT11 active metabolite); Sulfotaurolithocholic acid (STLC); and Temocaprilat. Exemplary inhibitors include Bacalin; Benzbromarone (non-competitive); BSO (Buthionine Sulfoximine); BSP; Cefodizime; CPT11 (irinotecan); Cyclosporin A; E3040; Folinic acid; Furosemide; Genistein; Glibenclamine; Glycyrrhizine; HIV protease inhibitors; Indocyanine Green; Indomethacin; LTC4; LTD4 receptor antagonist; Methotrexate; MK-571; Ouabaine; Probenecid; Quinidine; Rifampicin; S-decyl glutathione; Sulfinpyrazone; Taxol; Telmisaltan (BIBR 277) and its glucuronide metabolite; and Vinblastine.

Exemplary substrates of MRP3 that may be used in the practice of the present invention include DNP-S-glutathione; D-Glucuronide 17-b-estradiol; Glycocholate; LTC4; and Taurocholate. Exemplary inhibitors include methotrexate. A non-limiting example of a MRP6 substrate is BQ-123.

Verapamil is a well described L-type calcium channel blocker that also acts as a potent P-glycoprotein inhibitor. Another L-type calcium channel blocker, diltaizem, is a less reknowned P-glycoprotein inhibitor. Other P-glycoprotein inhibitors tested include the sodium channel blocker quinidine and the calmodulin antagonist thioridazine. MRP1 inhibitors tested include indomethacin and probenecid.

As would be appreciated by the skilled person, the concentration of substrate or inhibitor to use will vary depending on cell type and amount of ABC transporter expression on the cells. Offered as non-limiting examples, and with reference to CD34+ cells, verapamil concentrations from about 25 microg/mL to about 50 or about 75 microg/mL may be used; quinidine concentrations of about 50 microM to about 75 or about 100 microM may be used; diltiazem concentrations of about 100 microM to about 125, about 150, about 175 or about 200 microM may be used; ritonavir concentrations of about 5 to about 10, about 15 or about 20 microM may be used; vitamin E concentrations about 10 to about 20, about 30, about 40 or about 50 microM may be used; and probenecid concentrations of about 500 to about 1000, about 1500, or about 2000 microM may be used.

The skilled person would realize from the above that the amount or concentration of an ABC transporter substrate or inhibitor used in the practice of the invention will vary greatly depending on the substrate or inhibitor used. The amount or concentration that is effective to increase transduction as disclosed herein is referred to as the "effective amount", which is readily determined without undue experimentation. The "effective amount" may also be determined by the methods described herein by treating one cell or cells with one concentration of an ABC transporter substrate or inhibitor compound and treating another cell or cells in the absence of, or another concentration of, said compound (both with contact by a viral vector) followed by comparing the level of transduction in each case. The amount or concentration of a substrate or inhibitor compound that increases transduction can be determined by the simultaneous use of multiple amounts or concentrations of a compound or by the stepwise comparison of two amounts or concentrations of a compound.

The skilled person is also aware of the fact that for many inhibitors, as well as some substrates, higher doses tend to be toxic to the cells. Of course, the invention does not encompass conditions where transduction efficiency is enhanced but the cells are dead. The simple screening procedure to determ ine the amount or concentration of a substrate or inhibitor compound that increases transduction as provided herein may also detect toxicity to the cells as caused by the compound. The screening assay methods described above may also be used to identify the lowest effective amount or concentration of a compound that preserves the increase in transduction. This is particularly applicable to more potent, or cytotoxic, inhibitors, such as thioridazine and indomethacin, which may require lower concentrations to prevent cell death when used in the methods of the invention to increase transduction of viral vectors.

The invention may also take advantage of the observation that ABC transporter inhibitors (but not substrates) sodium vanadate (ATPase inhibitor), reserpine, and a neutralizing monoclonal antibody against ABCG2 did not increase transduction efficiency.

### Cell range

The invention may be practiced with any cell that is susceptible to transduction with a retroviral, preferably lentiviral, vector and that expresses an ABC transporter on its surface. Preferred cells are those of the human hemopoietic system, including hematopoietic stem cells. In some embodiments of the invention, the cells are preferably not hepatocytes.

Other preferred cells are those that express high levels of ABC transporters. The invention may thus also be practiced on SP cells, which are defined based on high-level expression of ABC transporters. Zhou et al. (Nat. Med. 7(9):1028-1034 2001) describe the expression of the ABC transporter Bcrpl/ABCG2 as a molecular determinant of the side-population (SP) phenotype. SP cells from a variety of tissues and organs may thus be used in the practice of the instant invention. The invention may also be practiced in stem cells of many organs, in addition to embryonic stem cells and adult totipotential stem cells.

For a cell in which the status of ABC transporter expression is unknown or where the expression of a particular ABC transporter is unknown, the ability of a potential substrate or inhibitor compound to increase transduction of said cell may be determined by the assay methods of the invention to see whether a compound increases transduction with a retroviral or lentiviral vector as described herein. This embodiment of the invention is particularly applicable in determining what ABC transporter substrate or inhibitor, as well as the amount or concentration of said substrate or inhibitor, to use with a particular primary cell in the practice of the present invention. Thus a particular cell type from a subject may be obtained and tested to determine the most effective substrate or inhibitor compound, as well as the effective amount thereof, to use in increasing transduction efficiency with a viral vector. The status of any ABC transporter expression in the cell may be known to assist in determining the range of substrates and inhibitors to use, but the status need not be known for the routine and repetitive assaying of various substrate or inhibitor compounds with a given cell type.

### Cell cytotoxicity

While exposure to verapamil resulted in cytotoxicity to the culture *in toto,* the CD34 positive cell content of the cultures was not reduced by verapamil during transduction of three sources (cord blood, peripheral blood, and bone marrow) of CD34 positive cells. The increase in the transduction level in SRC (SCID repopulating cells) compared to transplanted CD34 positive cells further suggests that verapamil mediated toxicity may selectively spare more primitive cells.

### Kits

Also provided are kits for use in the present invention, where such kits may comprise containers, each with one or more of the various reagents (typically in concentrated form) utilized in the methods, including, for example, buffers, culture media, viral vector, and ABC transporter substrate and inhibitor of the present invention. A label or indicator describing, or a set of instructions for use of, kit components in a transduction increasing method of the present invention, will also be typically included, where the instructions may be associated with a package insert and/or the packaging of the kit or the components thereof.

Having now generally described the invention, the same will be more readily understood through reference to the following examples which are provided by way of illustration, and are not intended to be limiting of the present invention, unless specified.

### Example I

### Materials and Methods Generally

The Examples herein were conducted generally as follows unless otherwise specified. Reagents: Verapamil (V4629), Diltaizem (D2521), Probenecid (P8761), Quinidine (Q0875), sodium orthovanadate (S6508) and reserpine (R0875) were purchased from Sigma Aldrich (Bedford, MA) as USP grade reagents when available. Ritonavir (Norvir) was obtained from Abbott Laboratories, Abbott Park, Illinois. The ABCG2 monoclonal antibody (clone 5D3) was purchased from ebioscience (San Diego, CA).

VRX494 is a safety modified HIV-1 based vector that encodes the eGFP gene and an antisense sequence against HIV-1 env from the HIV-1 LTR. Research grade vector was produced by calcium phosphate mediated transfection of vector plasmid with a VSV-G containing packaging construct into 293 cells. Supernatant was collected repeatedly 24 to 72 hours after transfection, pooled, and then concentrated by ultracentrifugation at 10,000 rpm for 12 hours. Clinical grade vector was packaged similarly in 293F cells under more stringent laboratory conditions. Supernatant was harvested at 24 hours and 36 hours after transfection. The supernatant is filtered through a series of cartridge filters of decreasing pore diameter, then concentrated by ultrafiltration. Benzonase is added by diafiltration to destroy contaminating plasmid and cellular DNA. The material is applied to a Sephacryl-500 gel filtration column then filtered through a 0.22-micron filter.

Cells: Frozen CD34 positive cells (greater than 98% purity) derived from adult bone marrow, adult G-CSF mobilized peripheral blood and cord blood were purchased from AllCells (Berkeley, CA). Additional peripheral blood CD34 cells mobilized by cyclophosphamide and GCSF and were generous gifts from Dr. Christian Chabannon.

Research grade vector transduction with VRX430: CD34+ cells were thawed according to manufacturer's specifications, then cultured at 37°C for 1 hour. Cells were cultured with an inhibitor at 37°C for 0 to 90 minutes before addition of vector. MOI 25 to 50 of vector was added to plates coated with 1.5 µg/cm² Retronectin (Takara, Japan), then cells were added to the plate and cultured at 37°C. Twenty-four hours after inhibitor addition, the cells were washed free of vector and drug and were resuspended in fresh medium containing MOI 25 of vector and cultured at 37°C. CD34+ cells were cultured in IMDM (Invitrogen, Carlsbad, CA) supplemented with 1x BIT (Stem Cell Technologies, Vancouver BC) and 25 to 100 ng/ml of TPO, Flt-3L and SCF (R&D Systems, Minneapolis, MN). Cells used in *in vitro* assays were washed free of vector four days after the start of transduction. Cells transplanted into mice were washed free of vector two days after the start of transduction and immediately transplanted into mice.

Cell culture: Four days after transduction CD34 positive cells were washed free of vector and maintained in liquid culture in IMDM plus 1x BIT supplemented with 25 ng/mL TPO, SCF, and Flt-3L, or plated in CFU and LTC-IC assays. Five hundred to 1000 cells were plated in methylcellulose (H3231, Stem Cell Technologies, Vancouver, BC) supplemented with 50 µg/mL SCF, 10 µg/mL IL3, 10 µg/mL GM-CSF and 1 U/mL EPO. Colony growth was scored two weeks after plating and was analyzed by flow cytometry for GFP expression. One to 3 x 10⁵ cells were overlayed upon MS-5 stromal cells for the LTC-IC assay. The cells underwent demidepopulation weekly, in which half of the medium was removed and replaced with fresh medium. The cells collected in each demidepopulation were analyzed by flow cytometry. After 5 weeks, a mix of adherent and non-adherent cells was plated into methylcellulose supplemented as above for formation of secondary CFU. Colonies were scored three weeks after plating and analyzed for GFP expression by flow cytometry.
Mice: NOD.CB17-Prkdcscid/J (formerly known as NODALtSz-Prkdcscid/J) mice were purchased from Jackson Laboratories (Bar Harbor, ME) and housed in microisolatorcages at Bioqual, Inc (Rockville, MD). All experiments were approved by the ACUC. 6 to 7 week old mice were sublethally irradiated with 350 cGy (¹³⁷Cs source), then transplanted the next day with 2 x 10⁵ cord blood CD34+ cells. Mice sacrificed at 6 to 8 weeks were tested for EGFP expression in CD45+, CD34+, CD33+, CD19+ and CD3+ subsets from the bone marrow using PE conjugated antibodies (clones HI30, 581, WM53, HIB19, UCHT1, respectively, Pharmingen, San Diego, CA) by flow cytometry (FACSCaliber, Becton Dickinson, Franklin Lakes, NJ). Mice sacrificed at 14 weeks were analyzed for EGFP expression in the CD45+, CD34+, CD33+, CD19+ and CD3+ subsets in the marrow, spleen and thymus. For secondary transplantation, half of the total marrow recovered from the primary mouse was transplanted into 350 cGy irradiated secondary recipient NOD.CB 17-Prkdcscid/J mice. Mice were sacrificed after 8 weeks and analyzed for EGFP expression in the CD45+, CD33+ and CD 19 subsets from the bone marrow.

### Example 2

### Verapamil increases transduction efficiency by clinical or research grade vectors

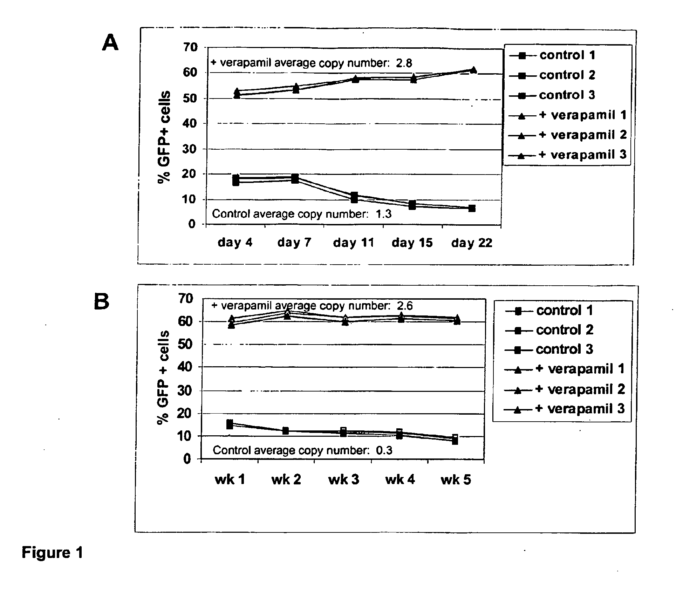

Hematopoietic stem cells (hsc) were transduced with clinical grade lentivirus vector expressing the marker gene green fluorescent protein (GFP) at a multiplicity of infection (MOI) of 25 according to vector titer as determined in Hela-tat cells. Transduction was performed in triplicate in the presence or absence of the ABC transporter inhibitor verapamil at a final concentration of 75 µg/ml. The percentage of GFP-expressing cells was measured in short-term (4-22 day) and long-term (1-5 weeks) cultures. In cultures where no verapamil was added, less than 20% of cells in cultures were transduced. However, addition of verapamil increased transduction to 50-60% in both short and long term cultures. In addition, the copy numbers per cell on average were about 2-fold higher in short term cultures transduced in the presence of verapamil and about 8-fold higher in long term cultures (Fig. 1, panels A and B, respectively).

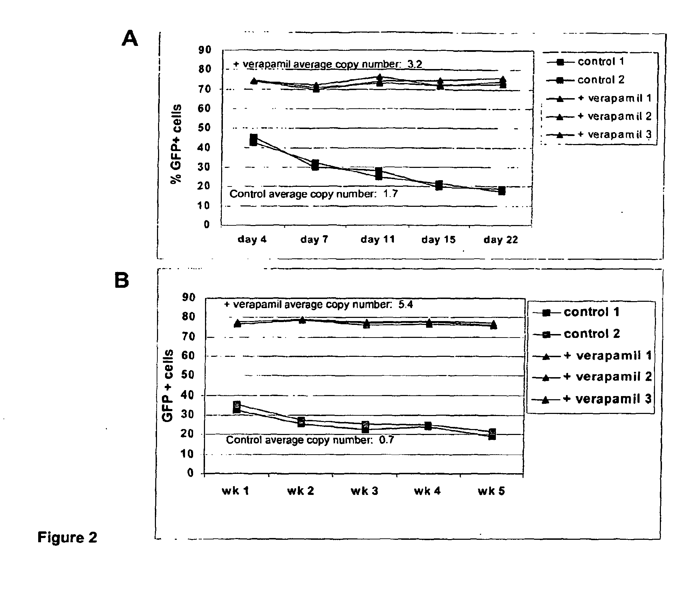

Parallel studies were performed using a research grade vector created at a very small scale using suboptimal conditions. Transduction was increased from 20-40% in short term cultures and 20-30% in long term cultures to 70-80% in both culture types with addition of verapamil. Similarly, copy numbers were increased 2-fold in short-term cultures and 8-fold in long-term cultures as observed before when using the clinical grade vector (Fig. 2, panels A and B, respectively).

### Example 3

### Verapamil increases transduction in stem cells

Verapamil increases transduction in stem cells isolated from mobilized peripheral blood, bone marrow, and cord blood. CD34+ cells were isolated from mobilized peripheral blood (mPB), bone marrow (BM), and cord blood (CB), and subsequently transduced at an MOI of 25 with clinical grade vector in the presence or absence of 75 µg/ml of verapamil. In addition, CD34+ cells were also transduced at an MOI of 20 with research grade vector in the presence and absence of verapamil. Addition of verapamil during transduction significantly increased the percentage of transduced cells in an 11 day culture, in colony forming units (CFU), in long term culture (LTC), and finally in secondary CFU which are cultured similarly to CFU except that they are derived from a 5 week LTC instead of fresh stem cells (see Table 1). The viability of exemplary cultures was assessed by propidium iodide staining and subsequent flow cytometric analysis. Addition of verapamil reduced the viability of tested stem cell cultures. Most sensitive to addition of verapamil were cultures isolated from peripheral blood, and least sensitive were those isolated from cord blood. It is of interest to note, that even significant drug-mediated toxicity, transduction was still significantly improved.

**Table 1**

| Effect of verapamil on percentage of stem cell transduction in various culture types | | | | | | | |
|---|---|---|---|---|---|---|---|
| | **CD34** | | | **CFU** | **LTC** | | **Secondary CFU** |
| | GFP | copy number | viability* | GFP | GFP | copy | GFP |
| mPB + verapamil RG | 75% | 3.2 | 70% | 58% | 78% | 5.4 | 56% |
| mPB - verapamil RG | 20% | 1.7 | 100% | 22% | 20% | 0.7 | 12% |
| mPB + verapamil | 60% | 4.2 | 68% | 23% | 61% | 2.6 | 42% |
| mPB - verapamil | 8% | 2.6 | 100% | 7% | 10% | 0.3 | 5% |
| BM + verapamil | 50% | | 80% | 37% | 45% | | 61% |
| BM - verapamil | 20% | | 100% | 28% | 22% | | 13% |
| CB + verapamil | 60% | | 91% | 38% | 50% | | 81% |
| CB - verapamil | 25% | | 100% | 20% | 30% | | 43% |
| | | | *relative to control | | | | |

### Example 4

### ABC transporter inhibitors are transporter substrates

ABC transporter inhibitors that are substrates increase transduction while those that are not substrates do not increase transduction. CD34+ cells from mobilized peripheral blood were transduced with clinical grade vector at an MOI of 25, in the presence or absence of the ABC transporter inhibitors verapamil (50 µg/ml) (as a positive control), diltiazem (100 µM), quinidine (50 µM), reserpine (25 µM), probenecid (1 µM), and anti-ABCG2 antibody (2 µg/ml). A significant increase in transduction efficiency was observed in stem cells transduced in the presence of verapamil, diltiazem, and quinidine. Drugs that did not increase transduction efficiency were reserpine, probenecid, and anti-ABCG2 antibody (Table 2). The drugs that were able to increase transduction were ABC transporter inhibitors that are substrates, and those that did not increase transduction were inhibitors that are not substrates. Reserpine, an ABC transporter inhibitor but not substrate, had no effect on transduction of CD34+ cells (data not shown). Probenecid, which inhibits the specific MRP2 subclass of ABC transporters which is structurally distinct, is a substrate inhibitor that did not increase transduction.

**Table 2**

| Comparison of ABC transporter substrates and inhibitors on transduction of stem cells | | | | | | | |
|---|---|---|---|---|---|---|---|
| | **CD34** | | | **CFU** | **LTC** | | **Secondary CFU** |
| | GFP | copy number | viability* | GFP | GFP | copy | GFP |
| mPB + verapamil | 30% | | 35% | 25% | 33% | | 49% |
| mPB + diltiazem | 19% | | 67% | 15% | 23% | | 26% |
| mPB + quinidine | 30% | | 43% | 24% | 33% | | 11% |
| mPB + reserpine | 3% | | 68% | 5% | 5% | | 3% |
| mPB + probenecid | 9% | | 83% | 8% | 11% | | 4% |
| mPB + anti-ABCG2 Ab | 4% | | 83% | 13% | 5% | | 1% |
| mPB no drugs added | 6% | | 100% | 5% | 8% | | 0% |
| | | | * relative to no drug control | | | | |

Transduction levels in this experiment were lower for the control verapamil-treated cultures than previously observed in Example 2. This is may be due to the decrease in viability observed after drug treatment in this experiment compared to the one described above (compare 35% viability relative to non-treated control cultures in Table 2 to 70% viability in Table 1).

### Example 5

### The HIV protease inhibitor ritonavir increases transduction efficiency

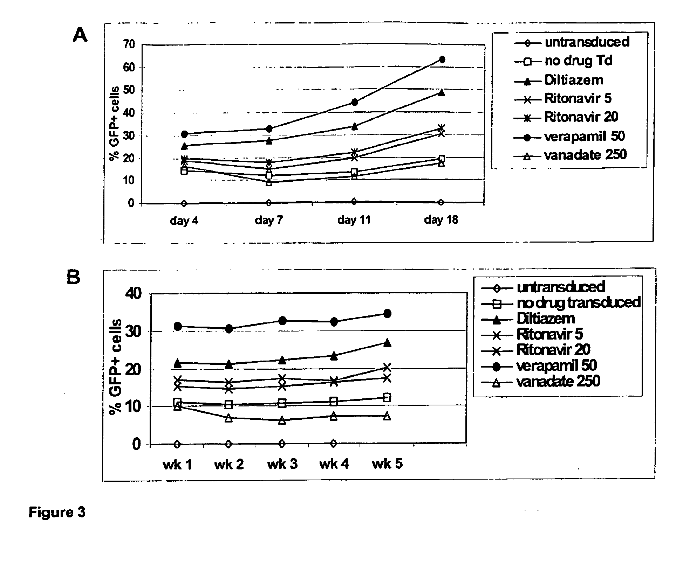

The HIV protease inhibitor, and P-gp substrate and inhibitor, ritonavir increases transduction efficiency in short and long term stem cell cultures. CD34+ cells isolated from mobilized peripheral blood were cultured short and long term. After one week, cultures were transduced in the presence or absence of the ABC-transporter inhibitors verapamil (50 µg/ml) (as a positive control), diltiazem (100 µM) (a drug with intermediate efficiency in increasing transduction, see Table 2), vanadate (250 µM) (an ABC transporter inhibitor that is not a substrate and therefore serves as a negative control; see Example 3) and ritonavir (at 5 µM or 20 µM). Ritonavir increased transduction efficiency over cells transduced without addition of drug. Addition of ritonavir during transduction did not increase the percentage of GFP-expressirig cells as well as diltiazem or verapamil. Treatment of cultures with vanadate during transduction reduced efficiency of gene transfer (Fig. 3, panels A and B).

### Example 6

### Maintenance of potential for differentiation

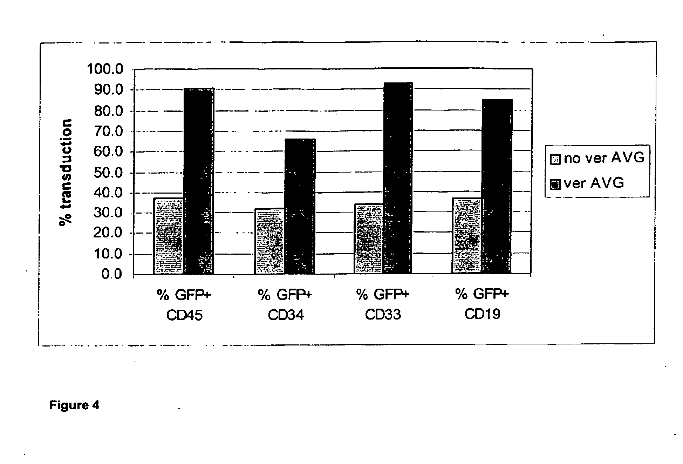

The increase in transduction efficiency in the presence of verapamil in stem cell repopulating cultures in the NOD-SCID xenotransplantation model is maintained 6-14 weeks post transfer in stem cells that have undergone differentiation. Hematopoietic stem cells (hsc) isolated from cord blood were transduced in the presence or absence of 50 and 75 µg/ml verapamil (no difference was observed between the two verapamil concentrations, so those data were pooled for Figure 4), and then injected into NOD-SCID mice. 6-8 weeks post-hsc transfer, bone marrow was isolated from mice and examined for GFP expression in cells bearing the human cell markers CD45, CD34, CD33, or CD19 (Figure 4). Cells transduced in the presence of verapamil retained the ability to repopulate and differentiate *in vivo* as reflected in the significantly higher percentage of GFP expressing cells isolated from mice transplanted with cells from that group. The percentage of transduced cells directly reflects that observed previously during in vitro culture of mobilized peripheral blood stem cells, and indicates that transduction of cells in the presence of verapamil does not alter the ability of these to repopulate and differentiate.

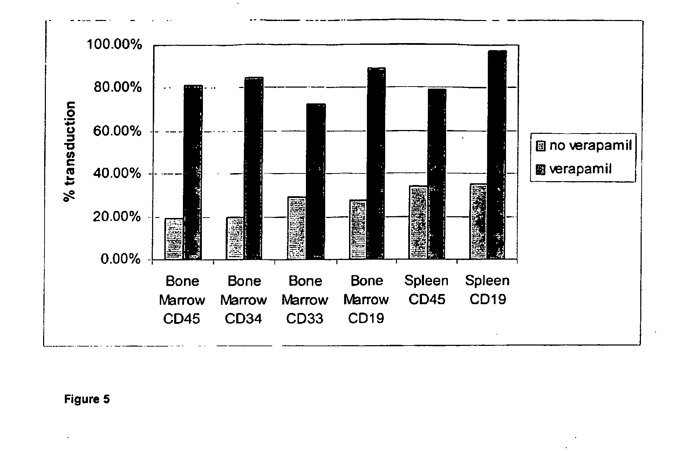

Fourteen weeks post-hsc transfer, cells were isolated from the bone marrow and spleen of xenotransplanted NOD-SCID mice. Again, CD45, CD34, CD33, and CD19 positive cells were measured for GFP expression in bone marrow cells. In addition, CD45 and CD19 positive cells isolated from the spleen were examined for GFP expression (Figure 5). A high percentage of GFP positive repopulating stem cells was observed in mice at 14 weeks, reflective of that observed at 6-8 weeks. CD19 and CD45 positive cells were also detected in the spleen, indicating that the stem cells injected into the mice were maturing into B cells and T cells. This population of cells retained an extremely high level of transduction, indicating that verapamil can increase transduction of an exogenous gene for expression in stem cells, which remain capable of high levels of expression of the gene after differentiation.

### Example 7

### Secondary transplantation in NOD-SCID mice

NOD-SCID mice were given a primary transplantation of stem cells isolated from cord blood in the presence of antibody to the murine IL-2 receptor beta chain to inhibit natural killer cell lysis of the transplanted cells. These mice exhibited on average 70% engraftment of human cells. Bone marrow was isolated at 14 weeks post transplantation. Total recovered cells (a mixture of human and mouse) were transplanted into naïve NOD/SCID recipient mice at 15-20 x 10⁶ cells per mouse. During secondary transplantation, mice were not given antibody to the murine IL-2 receptor due to inhibitory cost; therefore the percentage engraftment is much lower. Mice were sacrificed at 8 weeks post transplantation, and the percentage of GFP-positive human cells was assessed by flow cytometry (Table 3).

**Table 3**

| | | | | |
|---|---|---|---|---|
| Secondary transplantation of SRCs in NOD/SCID mice | | | | |

| **Bone Marrow** | **%CD45 Engraft.** | **%GFP CD45** | **%GFP CD19** | **%GFP CD33** |
|---|---|---|---|---|
| | | | | |
| Td no verapamil | <0.1% | 33% | 56% | 14% |
| Td no verapamil | 0.1% | 12% | 21% | 30% |
| Td no verapamil | 0.4% | 11% | 5% | 4% |
| | | | | |
| Td + verapamil | 0.3% | 97% | 98% | 51% |
| Td + vempamil | <0.1% | 59% | 100% | 38% |
| | | | | |
| No Td | 0.1% | 0% | 9% | 5% |
| No Td | <0.1% | 31% * | 0% | 22%* |
| No Td | 1.2% | 2% | 1% | 0% |

| | | | | |
|---|---|---|---|---|
| *Numbers resulted from flow cytometry artifact | | | | |

An increased percentage of GFP-positive cells was observed in mice transplanted with cells originally transduced in the presence of verapamil compared to those transduced without drug. This was the case in all cell lineages examined, which were CD45, CD19, and CD33. No detectable levels of CD34 were observed, consistent with differentiation of most or all of the naive human stem cells in the NOD/SCID mice. These data show that increased transduction in the presence of verapamil does not affect the ability of stem cells to repopulate and differentiate *in vivo,* and is therefore an effective tool for ensuring high levels of gene transfer and expression in stem cell transplantation.

## Claims

1. An *in vitro* or *ex vivo* method for increasing the transduction efficiency of an ATP-binding cassette (ABC) transporter expressing hematopoietic stem cell (HSC) with a vesicular stomatitis virus G (VSV-G) pseudotyped lentiviral vector, said method comprising:
(a) contacting said HSC with at least one compound comprising a substrate and an inhibitor of said ATP-binding cassette (ABC) transporter wherein said compound is not probenecid; and
(b) contacting said cell with said lentiviral vector.

2. The method of claim 1 wherein the substrate and inhibitor is in a soluble form.

3. The method of claim 1 wherein the substrate and inhibitor is in an immobilized form, immobilized on the surface of a viral vector particle or immobilized on a bead or vessel surface or on a wall of a tissue culture well, plate or bag.

4. The method of any one of claims 1 to 3 wherein the hematopoietic stem cell is derived from CD34+, CD34-, SP, KDR+ and ABCG2+ cell populations.

5. The method of claim 4 wherein said cell is a CD34+ cell, and is derived from cord blood, peripheral blood, mobilized peripheral blood or bone marrow.

6. The method of any one of the preceding claims wherein said substrate and inhibitor is selected from verapamil, diltiazem, cyclosporin A, PSC833, chloroquine, quinidine, saquinavir, nelfanir and ritonavir.

7. The method of any one of the preceding claims wherein said VSV-G pseudotyped lentiviral vector is derived from a human immunodeficiency virus (HIV), simian immunodeficiency virus (SIV), feline immunodeficiency virus (FIV), bovine immunodeficiency virus (BIV) or equine infectious anaemia virus (EIAV).

8. An *in vitro* or *ex vivo* method of determining the level of transduction efficiency of a vesicular stomatitis virus G (VSV-G) pseudotyped lentiviral vector in a haematopoietic stem cell (HSC) mediated by an ATP-binding cassette (ABC) transporter substrate and inhibitor compound, said method comprising
(a) contacting one or more cells of a population of said HSC with said VSV-G pseudotyped lentiviral vector in the presence of said compound to produce a first contacted cell or population of cells and contacting a second cell or cells of said population with said VSV-G pseudotyped lentiviral vector in the absence of said compound to produce a second contacted cell or population of cells; and
(b) determining the level of transduction efficiency with said lentiviral vector in said first contacted cell or population of cells and in said second contacted cell or population of cells.

9. The method of claim 8 wherein said population of cells are primary cells.

10. The method of claim 8 wherein said cells are from or derived from a human being.

11. A composition for increasing transduction efficiency of an ATP-binding cassette (ABC) transporter expressing haematopoietic stem cell (HSC) with a vesicular stomatitis virus G (VSV-G) pseudotyped lentiviral vector, said composition comprising said vesicular stomatitis virus G (VSV-G) pseudotyped lentiviral vector and a substrate and an inhibitor of an ABC transporter, wherein said substrate and inhibitor is not probenecid.

12. The composition according to claim 11 wherein said substrate and inhibitor is selected from verapamil, diltiazem, cyclosporin A, PSC833, chloroquine, quinidine, saquinavir, nelfanir and ritonavir.

13. The composition according to any one of claims 11 to 12 wherein said vector is as defined in claim 7.

14. The composition according to any one of claims 11 to 13 wherein said cell is as defined in claim 4 or 5.

15. A kit for increasing transduction efficiency of an ATP-binding cassette (ABC) transporter expressing haematopoietic stem cell (HSC) with a vesicular stomatitis virus G (VSV-G) pseudotyped lentiviral vector, said kit comprising said vesicular stomatitis virus G (VSV-G) pseudotyped lentiviral vector and an ATP-binding cassette (ABC) transporter substrate and inhibitor compound, wherein said compound is not probenecid.

16. The method of claim 1 wherein the substrate and inhibitor of the ABC transporter is cultured with said HSC before transduction with the VSV-G pseudotyped lentiviral vector.

17. The method of claim 1 wherein the substrate and inhibitor of the ABC transporter is added to said HSC simultaneously with the VSV-G pseudotyped lentiviral vector.

18. The method of claim 2 wherein the substrate and inhibitor is cross-linked by other molecules.

19. The kit of claim 15 further comprising a set of instructions for practicing the method of any one of claims 1 to 7.

## Patentansprüche

1. *In-vitro-* oder *Ex-vivo*-Verfahren zur Erhöhung der Transduktionseffizienz einer ATP-bindende-Kassette(ABC)-Transporter exprimierenden hämopoetischen Stammzelle (HSC) mit einem Vesikuläres Stomatitis-Virus G (VSV-G)-pseudotypisierten lentiviralen Vektor, wobei das Verfahren folgendes umfasst:
(a) Inkontaktbringen der HSC mit mindestens einer Verbindung, die ein Substrat(stoff) und einen Hemmstoff bzw. Inhibitor des ATP-bindende-Kassette-(ABC)-Transporters umfasst, wobei es sich bei der Verbindung nicht um Probenecid handelt; und
(b) Inkontaktbringen der Zelle mit dem lentiviralen Vektor.

2. Verfahren von Anspruch 1, wobei der Substrat- und Hemmstoff in einer löslichen Form vorliegt.

3. Verfahren von Anspruch 1, wobei der Substrat- und Hemmstoff in einer immobilisierten Form, immobilisiert auf der Oberfläche eines viralen Vektorpartikels oder immobilisiert auf einer Kügelchen- oder Gefäßoberfläche oder auf der Wand einer Gewebekultur-Vertiefung, -Platte oder eines Beutels vorliegt.

4. Verfahren von einem beliebigen der Ansprüche 1 bis 3, wobei die hämopoetische Stammzelle aus CD34⁺-, CD34⁻-, SP-, KDR⁺- und ABCG2⁺-Zellpopulationen abgeleitet ist.

5. Verfahren von Anspruch 4, wobei die Zelle eine CD34⁺-Zelle ist und aus Nabelschnurblut, peripherem Blut, mobilisiertem peripherem Blut oder Knochenmark abgeleitet ist.

6. Verfahren von einem beliebigen der vorangehenden Ansprüche, wobei der Substrat- und Hemmstoff aus Verapamil, Diltiazem, Cyclosporin A, PSC833, Chloroquin, Quinidin, Saquinavir, Nelfanir und Ritonavir gewählt ist.

7. Verfahren von einem beliebigen der vorangehenden Ansprüche, wobei der VSV-G-pseudotypisierte lentivirale Vektor aus einem Humanen Immunschwäche-Virus (HIV), Affen-Immunschwäche-Virus (SIV), Felinem Immunschwäche-Virus (FIV), Rinder-Immunschwäche-Virus (BIV) oder Equinen-Infektiösen-Anämie-Virus (EIAV) abgeleitet ist.

8. *In-vitro-* oder *Ex-vivo*-Verfahren zur Bestimmung des Spiegels der Transduktionseffizienz eines Vesikuläres Stomatitis-Virus G (VSV-G)-pseudotypisierten lentiviralen Vektors in einer hämopoetischen Stammzelle (HSC), die durch eine ATP-bindende-Kassette(ABC)-Transporter-Substrat-und-Hemmstoff-Verbindung vermittelt wird, wobei das Verfahren folgendes umfasst:
(a) Inkontaktbringen einer oder mehrer Zellen einer Population der HSC mit dem VSV-G-pseudotypisierten lentiviralen Vektor in Gegenwart der Verbindung zur Erzeugung einer ersten kontaktierten Zelle oder Population von Zellen, und Inkontaktbringen von einer zweiten Zelle oder Zellen der Population mit dem VSV-G-pseudotypisierten lentiviralen Vektor in Abwesenheit der Verbindung zur Erzeugung einer zweiten kontaktierten Zelle oder Population von Zellen; und
(b) Bestimmen des Spiegels der Transduktionseffizienz mit dem lentiviralen Vektor in der ersten kontaktierten Zelle oder Population von Zellen und in der zweiten kontaktierten Zelle oder Population von Zellen.

9. Verfahren von Anspruch 8, wobei es sich bei der Population von Zellen um primäre Zellen handelt.

10. Verfahren von Anspruch 8, wobei die Zellen aus einem Menschen stammen oder abgeleitet sind.

11. Zusammensetzung zur Erhöhung der Transduktionseffizienz einer ATP-bindende-Kassette(ABC)-Transporter exprimierenden hämopoetischen Stammzelle (HSC) mit einem Vesikuläres Stomatitis-Virus G (VSV-G)-pseudotypisierten lentiviralen Vektor, wobei die Zusammensetzung den Vesikuläres Stomatitis-Virus G (VSV-G)-pseudotypisierten lentiviralen Vektor und ein Substrat und einen Hemmstoff eines ABC-Transporters umfasst, wobei es sich bei dem Substrat- und Hemmstoff nicht um Probenecid handelt.

12. Zusammensetzung gemäß Anspruch 11, wobei der Substrat- und Hemmstoff aus Verapamil, Diltiazem, Cyclosporin A, PSC833, Chloroquin, Quinidin, Saquinavir, Nelfanir und Ritonavir gewählt ist.

13. Zusammensetzung gemäß einem beliebigen der Ansprüche 11 bis 12, wobei der Vektor wie in Anspruch 7 definiert ist.

14. Zusammensetzung gemäß einem beliebigen der Ansprüche 11 bis 13, wobei die Zelle wie in Anspruch 4 oder 5 definiert ist.

15. Kit zur Erhöhung der Transduktionseffizienz einer ATP-bindende-Kassette(ABC)-Transporter exprimierenden hämopoetischen Stammzelle (HSC) mit einem Vesikuläres Stomatitis-Virus G (VSV-G)-pseudotypisierten lentiviralen Vektor, wobei das Kit den Vesikuläres Stomatitis-Virus G (VSV-G)-pseudotypisierten lentiviralen Vektor und eine ATP-bindende-Kassette(ABC)-Transporter-Substrat-und-Hemmstoff-Verbindung umfasst, wobei es sich bei der Verbindung nicht um Probenecid handelt.

16. Verfahren von Anspruch 1, wobei man den Substrat- und Hemmstoff des ABC-Transporters mit der HSC vor der Transduktion mit dem VSV-G-pseudotypisierten lentiviralen Vektor kultiviert.

17. Verfahren von Anspruch 1, wobei man den Substrat- und Hemmstoff des ABC-Transporters der HSC gleichzeitig mit dem VSV-G-pseudotypisierten lentiviralen Vektor zusetzt.

18. Verfahren von Anspruch 2, wobei der Substrat- und Hemmstoff durch andere Moleküle vernetzt ist.

19. Kit von Anspruch 15, das ferner einen Satz von Anweisungen zur Ausführung des Verfahrens von einem beliebigen der Ansprüche 1 bis 7 umfasst.

## Revendications

1. Procédé *in vitro* ou *ex vivo* pour améliorer l'efficacité de transduction d'une cellule souche hématopoïétique (CSH) exprimant un transporteur d'une cassette de liaison à l'ATP (ABC) avec un vecteur lentiviral pseudotypé par la protéine G du virus de la stomatite vésiculaire (VSV-G), ledit procédé comprenant :
(a) la mise en contact de ladite CSH avec au moins un composé comprenant un substrat et un inhibiteur dudit transporteur d'une cassette de liaison à l'ATP (ABC), dans lequel ledit composé n'est pas le probénécide ; et
(b) la mise en contact de ladite cellule avec ledit vecteur lentiviral.

2. Procédé selon la revendication 1, dans lequel le substrat et inhibiteur est sous une forme soluble.

3. Procédé selon la revendication 1, dans lequel le substrat et inhibiteur est sous une forme immobilisée, immobilisé sur la surface d'une particule de vecteur viral ou immobilisé sur une bille ou la surface d'un récipient ou sur une paroi d'un puit, d'une plaque ou d'une poche de culture tissulaire.

4. Procédé selon l'une quelconque des revendications 1 à 3, dans lequel la cellule souche hématopoïétique est dérivée de populations cellulaires CD34+, CD34-, SP, KDR+ et ABCG2+.

5. Procédé selon la revendication 4, dans lequel ladite cellule est une cellule CD34+, et est dérivée de sang du cordon, de sang périphérique, de sang périphérique mobilisé ou de moelle osseuse.

6. Procédé selon l'une quelconque des revendications précédentes, dans lequel ledit substrat et inhibiteur est sélectionné parmi le vérapamil, le diltiazem, la cyclosporine A, PSC833, la chloroquine, la quinidine, le saquinavir, le nelfanir et le ritonavir.

7. Procédé selon l'une quelconque des revendications précédentes, dans lequel ledit vecteur lentiviral pseudotypé par la VSV-G est dérivé d'un virus de l'immunodéficience humaine (VIH), d'un virus de l'immunodéficience simienne (VIS), d'un virus de l'immunodéficience féline (VIF), d'un virus de l'immunodéficience bovine (VIB) ou d'un virus de l'anémie infectieuse équine (EIAV).

8. Procédé *in vitro* ou ex *vivo* pour déterminer le niveau d'efficacité de transduction d'un vecteur lentiviral pseudotypé par la protéine G du virus de la stomatite vésiculaire (VSV-G) dans une cellule souche hématopoïétique (CSH), médiée par un composé substrat et inhibiteur d'un transporteur d'une cassette de liaison à l'ATP (ABC), ledit procédé comprenant
(a) la mise en contact d'une ou de plusieurs cellules d'une population de ladite CSH avec ledit vecteur lentiviral pseudotypé par la VSV-G en présence dudit composé afin de produire une première cellule ou population de cellules en contact, et la mise en contact d'une seconde cellule ou de cellules de ladite population avec ledit vecteur lentiviral pseudotypé par la VSV-G en l'absence dudit composé afin de produire une seconde cellule ou population de cellules en contact ; et
(b) la détermination du niveau de l'efficacité de transduction avec ledit vecteur lentiviral dans ladite première cellule ou population de cellules en contact et dans ladite seconde cellule ou population de cellules en contact.

9. Procédé selon la revendication 8, dans lequel ladite population de cellules sont des cellules primaires.

10. Procédé selon la revendication 8, dans lequel lesdites cellules sont issues ou sont dérivées d'un être humain.

11. Composition pour améliorer l'efficacité de transduction d'une cellule souche hématopoïétique (CSH) exprimant un transporteur d'une cassette de liaison à l'ATP (ABC) avec un vecteur lentiviral pseudotypé par la protéine G du virus de la stomatite vésiculaire (VSV-G), ladite composition comprenant ledit vecteur lentiviral pseudotypé par la protéine G du virus de la stomatite vésiculaire (VSV-G) et un substrat et un inhibiteur d'un transporteur ABC, dans laquelle ledit substrat et inhibiteur n'est pas le probénécide.

12. Composition selon la revendication 11, dans laquelle ledit substrat et inhibiteur est sélectionné parmi le vérapamil, le diltiazem, la cyclosporine A, PSC833, la chloroquine, la quinidine, le saquinavir, le nelfanir et le ritonavir.

13. Composition selon l'une quelconque des revendications 11 à 12, dans laquelle ledit vecteur est tel que défini dans la revendication 7.

14. Composition selon l'une quelconque des revendications 11 à 13, dans laquelle ladite cellule est telle que définie dans la revendication 4 ou 5.

15. Trousse pour améliorer l'efficacité de transduction d'une cellule souche hématopoïétique (CSH) exprimant un transporteur d'une cassette de liaison à l'ATP (ABC) avec un vecteur lentiviral pseudotypé par la protéine G du virus de la stomatite vésiculaire (VSV-G), ladite trousse comprenant ledit vecteur lentiviral pseudotypé par la protéine G du virus de la stomatite vésiculaire (VSV-G) et un composé substrat et inhibiteur d'un transporteur d'une cassette de liaison à l'ATP (ABC), dans laquelle ledit composé n'est pas le probénécide.

16. Procédé selon la revendication 1, dans lequel le substrat et inhibiteur du transporteur ABC est cultivé avec ladite CSH avant la transduction avec le vecteur lentiviral pseudotypé par la VSV-G.

17. Procédé selon la revendication 1, dans lequel le substrat et inhibiteur du transporteur ABC est ajouté à ladite CSH simultanément avec le vecteur lentiviral pseudotypé par la VSV-G.

18. Procédé selon la revendication 2, dans lequel le substrat et l'inhibiteur est réticulé par d'autres molécules.

19. Trousse selon la revendication 15, comprenant en outre un ensemble d'instructions pour mettre en oeuvre le procédé selon l'une quelconque des revendications 1 à 7.
